# EUROPÄISCHE PATENTSCHRIFT

(11) **EP 2 066 380 B1**
(45) Veröffentlichungstag und Bekanntmachung des Hinweises auf die Patenterteilung: **09.10.2019**
(21) Anmeldenummer: 07801718.3
(22) Anmeldetag: 17.08.2007
(51) Int. Cl.: A61M 15/00

(54) **INHALATOR**
INHALER
INHALATEUR

(30) Priorität: 20.09.2006 DE 102006044756
(43) Veröffentlichungstag der Anmeldung: 10.06.2009
(73) Patentinhaber: Boehringer Ingelheim International GmbH, 55216 Ingelheim (DE)
(72) Erfinder: KLADDERS, Heinrich, 45468 Muelheim-Ruhr (DE)
(74) Vertreter: Simon, Elke Anna Maria
(86) Internationale Anmeldenummer: PCT/EP2007/007268
(87) Internationale Veröffentlichungsnummer: WO 2008/034504

(56) Entgegenhaltungen:
- WO-A-00/53248
- WO-A-02/11894
- WO-A-02/45783
- WO-A-02/053215
- WO-A1-95/28980
- WO-A1-97/05918
- GB-A- 2 279 273
- US-A- 5 740 793
- US-A- 6 012 454
- US-A1- 2006 118 107

## Beschreibung

Die vorliegende Erfindung betrifft einen Inhalator gemäß dem Oberbegriff des Anspruchs 1 oder 7 zur Ausgabe und insbesondere Zerstäubung einer vorzugsweise pulverförmigen Formulierung von einem Träger.

Die vorliegende Erfindung betrifft insbesondere einen Inhalator zur Ausgabe bzw. Inhalation einer vorzugsweise pulverförmigen Formulierung, also einen Pulverinhalator. Jedoch kann die Formulierung grundsätzlich auch in flüssiger Phase, als Dispersion oder in sonstiger fluidisierbarer Form vorliegen.

Bei der Formulierung handelt es sich insbesondere um ein therapeutisches Mittel bzw. Arzneimittel. Insbesondere enthält die Formulierung dementsprechend mindestens einen Wirkstoff oder besteht daraus. Die Formulierung dient also insbesondere der medizinischen Behandlung oder sonstigen therapeutischen Zwecken.

Bei der vorliegenden Erfindung ist die Formulierung in oder von einem Träger aufgenommen, insbesondere vordosiert in einzelnen Dosen.

Die EP 0 147 755 A2 offenbart einen Inhalator für die Inhalation pulverförmiger Arzneimittel aus länglichen Kapseln. Der Inhalator weist eine Kapselkammer auf, in die jeweils eine Kapsel manuell einführbar ist. Die Kapsel wird durch manuelle Betätigung einer Öffnungseinrichtung in der Kapselkammer längsseitig angestochen und dadurch geöffnet. Beim Inhalieren führt ein durch die Kapselkammer strömender Luftstrom dazu, daß die Kapsel in der Kapselkammer hin- und herbewegt wird, wobei das pulverförmige Arzneimittel ausgetragen und im Luftstrom dispergiert wird.

Die US 5740793 offenbart einen Trockenpulverinhalator, in dem das Medikament in Bereichen vordefinierter Größe auf einem streifenförmiger Träger angeordnet ist. Diese Bereiche werden sequentiell einer Kammer mit Lufteinlässen zugeführt, aus der ein Patient über ein Mundrohr das Medikament über den bei Einatmung erzeugten Luftstrom einatmen kann. Der Inhalator kann zur Unterstützung der Medikamentenfreisetzung ein Impaktionsmittel wie einen Hammer, eine Kratzvorrichtung, Druckluft oder einen piezoelektrischen / elektromagnetischen / rotationsgetriebenen Vibrator aufweisen. Die US 6012454 zeigt ein ähnliches Gerät, bei dem atemzugsgesteuert das Impaktionsmittel elektrisch angetrieben wird.

Die WO 02/053215 A2 offenbart einen Trockenpulverinhalator mit einer Einzeldosisvorratskammer, bei der sich ein Ausgabekanal getrennt durch eine flexible und biegbare Dichtungsplatte an die Vorratskammer anschließt. Wenn Druckluft auf die Dosis geleitet wird, wird die Dichtungsplatte weggebogen, so dass das Pulver in und durch den Ausgabekanal getrieben wird. Der Luftstrom verursacht eine Vibration der Dichtungsplatte im Ausgabekanal, wodurch die Partikel im Ausgabekanal zerkleinert werden. Die GB 2279273 offenbart ein Zerstäubungsprinzip, bei dem ein Pulver auf ein Diaphragma gegeben wird, das unter Einwirkung von Druckluftpulsen vibriert und so zur Deagglommeration der ausgebrachten Partikel beiträgt. Die WO 02/11894 A1 offenbart einen Pulverinhalator mit einem Diaphragmaventil, das bei einatmungsinduzierten Druckschwankungen walkt und darauf befindliches Medikament aus dem Inhalator auswirft.

Die WO 95/28980 A1 offenbart einen Pulverinhalator mit einer Druckluftpumpe. Über ein Ventil ist die Pumpe an einen in eine Auslassdüse mündenden Entladungskanal angeschlossen, in dem eine Dosiskammer mit einem flexiblen Insert angeordnet ist.

Die WO 97/05918 A2 offenbart einen Pulverinhalator, bei dem das Pulver als Einzeldosis in einer auf einer Platte ausgeformten Kavität eingesetzt wird. Die Platte ist vor Benutzung beidseitig über Abziehfolien versiegelt. Das Pulver wird durch einen über Einatmung am Mundstück erzeugten Luftstrom aus der Kavität an deagglommerierend wirkenden Prallplatten vorbei zum Mundstück transportiert.

Die WO 00/53248 A1 zeigt als Detail in einem Pulverinhalator einen Dosisschutz in Form einer flexiblen Lasche, die eine Dosis-Kavität in einem Strömungskanal abdeckt. Nur bei Vorliegen einer Luftströmung in eine bestimmte Richtung (d.h. beim Einatmen des Patienten) wird die Lasche durch die Strömung angehoben, so dass die Dosis in der Strömung freigesetzt wird. Ohne Luftströmung bzw. bei gegenläufiger Luftströmung (d.h. bei Ausatmung) bleibt die Kavität verschlossen.

Der vorliegenden Erfindung liegt die Aufgabe zugrunde, einen neuen Inhalator anzugeben, so daß auf einfache Weise eine effektive Ausbringung und insbesondere Zerstäubung einer insbesondere pulverförmigen Formulierung ermöglicht wird.

Die obige Aufgabe wird durch einen Inhalator gemäß Anspruch 1 oder 7 gelöst. Vorteilhafte Weiterbildungen sind Gegenstand der Unteransprüche.

Ein erster Aspekt der vorliegenden Erfindung liegt darin, daß ein Träger oder Förderkanal für eine zu fördernde, anzugebende und/oder zu dispergierende Formulierung flexibel, flach, bandförmig, streifenförmig, fadenartig, blisterartig und/oder folienartig ausgebildet ist, wobei der Träger bzw. Förderkanal durch einen Luftstrom zum Austrag der Formulierung bewegbar, beispielsweise in Schwingung versetzbar, ist. Die Formulierung kann so besonders wirkungsvoll insbesondere aus einer entsprechenden Aufnahme des Trägers ausgegeben, dispergiert und/oder ausgetragen werden.

Zum Austrag der Formulierung kann wahlweise der Luftstrom zum Bewegen des Trägers bzw. Förderkanals oder ein sonstiger Luftstrom eingesetzt werden.

Ein weiterer, auch unabhängig von der konkreten Gestaltung des Trägers bzw. Förderkanals realisierbarer Aspekt der vorliegenden Erfindung liegt darin, ein vom Träger bzw. Förderkanal getrenntes Element vorzusehen, das von dem Luftstrom in Bewegung, insbesondere in Schwingung, versetzt wird, um den Träger bzw. Förderkanal zur Ausgabe bzw. zum Austrag bzw. zum Dispergieren der Formulierung zu bewegen. Beispielsweise kann das Element als Kugel bzw. Schlagkörper ausgebildet sein.

Bei der vorliegenden Erfindung sind die Begriffe "Luft" und "Luftstrom" vorzugsweise in einem weiteren Sinne auch dahingehend zu verstehen, daß auch ein sonstiges Gas bzw. eine Strömung eines sonstigen Gases umfaßt ist. Nachfolgend wird jedoch immer der Begriff "Luft" verwendet, da üblicherweise Luft als Gas zum Antrieb bzw. zur Bewegung und/oder als Fördermedium zur Förderung der Formulierung nach dem Lösen von oder aus dem Träger bzw. zum Dispergieren der Formulierung eingesetzt wird.

Gemäß einem weiteren, auch unabhängig realisierbaren Aspekt der vorliegenden Erfindung weist der Inhalator eine Pumpe mit einem teleskopierbaren Pumpraum zum Erzeugen eines Luftstroms auf. Dies gestattet insbesondere die Erzeugung eines relativ großen Luftstroms bzw. einer großen Fördermenge pro Pumpenhub und damit auf einfache Weise eine effektive Ausbringung und insbesondere Zerstäubung einer vorzugsweise pulverförmigen Formulierung. Im Transportzustand kann der Pumpraum zusammengeschoben werden, so daß der Inhalator besonders wenig Raum beim Transport, beispielsweise in einer Tasche, einnimmt.

Einzelne Aspekte, Merkmale, Eigenschaften und Vorteile der vorliegenden Erfindung ergeben sich aus den Ansprüchen und der folgenden Beschreibung bevorzugter Ausführungsformen anhand der Zeichnung. Es zeigt:
- Fig. 1: einen schematischen Schnitt eines Inhalators gemäß einer ersten Ausführungsform;
- Fig. 2: einen schematischen Schnitt eines Inhalators gemäß einer zweiten Ausführungsform;
- Fig. 3: einen schematischen Schnitt eines Inhalators ;

- Fig. 4: eine schematische Draufsicht eines Trägers für einen vorschlagsgemäßen Inhalator;
- Fig. 5: einen schematischen Schnitt eines Inhalators ; und

- Fig. 6: einen schematischen Schnitt eines Inhalators gemäß einer weiteren Ausführungsform.

In den Figuren werden für gleiche oder ähnliche Teile die gleichen Bezugszeichen verwendet, auch wenn eine wiederholte Beschreibung weggelassen ist. Insbesondere ergeben sich dann auch die gleichen oder entsprechende Vorteile und Eigenschaften. Die einzelnen Figuren sind aus Darstellungs- oder Vereinfachungsgründen nicht maßstabsgerecht und auf wesentliche für die Erfindung relevante Komponenten reduziert.

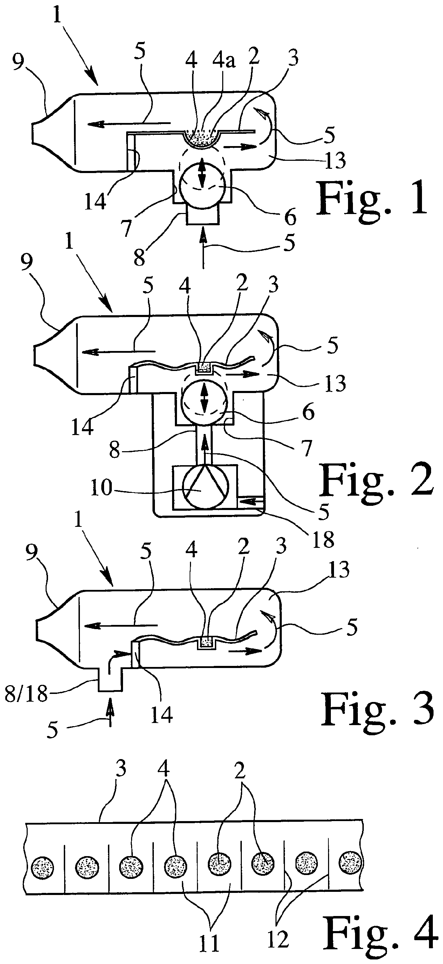

Fig. 1 zeigt schematisch den Aufbau eines vorschlagsgemäßen Inhalators 1 gemäß einer Ausführungsform. Der Inhalator 1 ist vorzugsweise tragbar ausgebildet und/oder arbeitet insbesondere nur mechanisch.

Der Inhalator 1 dient der Ausgabe bzw. Inhalation einer vorzugsweise pulverförmigen Formulierung 2 im eingangs genannten Sinne. Die Formulierung 2 ist vorzugsweise vordosiert in einer einzelnen Dosis oder mehrere Dosen, die vorzugsweise nacheinander und unabhängig voneinander ausgebbar sind.

Beim Darstellungsbeispiel ist die Formulierung 2 vorzugsweise vordosiert, also außerhalb des Inhalators 1 bereits in einer einzelnen Dosis oder in mehrere Dosen vordosiert. Grundsätzlich ist es jedoch auch möglich, die Formulierung 2 erst unmittelbar vor jeder Inhalation und/oder im Inahlator 1 zu dosieren.

Ein Träger 3 trägt die Formulierung 2 bzw. stellt diese bereit. Insbesondere weist der Träger 3 mindestens eine Vertiefung bzw. Aufnahme 4 o. dgl. zur Aufnahme insbesondere einer Dosis der Formulierung 2 auf. Jedoch sind hier auch andere konstruktive Lösungen möglich. Beispielsweise kann die Formulierung 2 auf der Oberfläche oder in einem Bereich der Oberfläche oder auf der gesamten Oberfläche des Trägers 3 angeordnet oder verteilt sein. Weiter sind hier auch sonstige Konfigurationen möglich. Insbesondere kann der Träger 3 auch als Behältnis, Kammer, Hülle o. dgl. ausgebildet sein.

Der Träger 3 ist bei der ersten Ausführungsform insbesondere flexibel, flach, bandförmig, streifenförmig, blisterartig und/oder folienartig ausgebildet. Es ist aus einem geeigneten Material, insbesondere aus Kunststoff, Metallfolie, einem Verbundmaterial o. dgl., hergestellt.

Der Träger 3 kann nur eine Aufnahme 4 oder mehrer Aufnahmen 4 aufweisen. Jede Aufnahme 4 kann bedarfsweise von einer optionalen Abdeckung 4a, wie in Figur 1 beispielhaft angedeutet, abgedeckt sein. Zum Austrag der Formulierung 2 ist die Abdeckung 4a dann vorzugsweise öffenbar, abziehbar oder dergleichen. Beispielsweise kann die Abdeckung 4a aufgeschnitten, aufgerissen, perforiert oder in sonstiger Weise zumindest teilweise entfernt und/oder geöffnet werden, um einen Austrag der Formulierung 2 zu ermöglichen. Besonders bevorzugt kann das Öffnen auch durch eine Bewegung des Trägers 3, auf die später noch eingegangen wird, erfolgen. Die Abdeckung 4a und/oder eine verhältnismäßig kleine Austrittsöffnung oder dergleichen führt bzw. führen besonders bevorzugt dazu, daß die Formulierung 2 verhältnismäßig langsam - insbesondere mit einer gewünschten Rate - abgegeben und ausgetragen wird.

Der Träger 3 ist mittels eines Luftstroms 5 zum Austrag der Formulierung 2 - also zum Lösen der Formulierung 2 vom Träger 3 bzw. aus der Aufnahme 4 - bewegbar, insbesondere in Schwingung versetzbar. Der Luftstrom 5 kann unmittelbar und/oder mittelbar auf den Träger 3 einwirken bzw. diesen bewegen.

Bei der ersten Ausführungsform wirkt der Luftstrom 5 insbesondere mittelbar auf den Träger 3 ein. Der Inhalator 1 weist hierzu ein vom Träger 3 getrenntes Element 6 auf, das vom Luftstrom 5 zur Bewegung des Trägers 3 bewegbar ist. Insbesondere ist das Element 6 vom Luftstrom 5 in Schwingung versetzbar.

Beim Darstellungsbeispiel ist das Element 6 vorzugsweise im wesentlichen kugelförmig ausgebildet. Es kann jedoch auch jede sonstige geeignete Form, beispielsweise eine längliche, eiförmige oder rotationsymmetrische Form aufweisen.

Vorzugsweise ist das Element 6 in einer Kammer 7 aufgenommen. Der Kammer 7 ist über einen Zuführkanal 8 der Luftstrom 5 zuführbar. Der Zuführkanal 8 öffnet sich zur Kammer 7 insbesondere mit einem gegenüber der Kammer 7 und insbesondere gegenüber dem Element 6 verringerten Querschnitt. Jedoch sind hier auch anderen Anordnungen möglich.

Bei entsprechender Dimensionierung bzw. Abstimmung wird das Element 6 vom Luftstrom 5 hin- und herbewegt und insbesondere in Schwingung versetzt, und zwar besonders bevorzugt längs der Hauptströmungsrichtung. Besonders bevorzugt entsprechen die geometrischen Verhältnisse den Angaben in der EP 0 147 755 A2.

Insbesondere wird die hin- und hergehende Bewegung des Elements 6 durch den sogenannten Bernoulli-Effekt bewirkt.

Das Element 6 trifft insbesondere quer zur Längs- bzw. Flächenerstreckung auf den Träger 3, besonders bevorzugt auf der der Formulierung 2 abgewandten Trägerseite. Der Träger 2 wird durch die Bewegung des Elements 6 dann entsprechend in eine vorzugsweise flatterartige oder wellenartige Bewegung bzw. Schwingung versetzt. Dies führt auf sehr effektive Weise zu der gewünschten Bewegung des Trägers 3, um ein Dispergieren und Lösen der Formulierung 2 vom Träger 3 bzw. aus einer zugeordneten Aufnahme 4 o. dgl. zu unterstützen bzw. zu erreichen. Weiter kann der Träger 3 bzw. die Aufnahme 4 durch die Bewegung ggf. auch geöffnet, beispielsweise aufgerissen, werden.

Besonders bevorzugt wirkt das Element 6 schlagend auf den Träger 3 ein. In diesem Fall bildet das Element 6 einen Schlagkörper. Jedoch können hier auch andere Wirkmechanismen zum Tragen kommen.

Vorzugsweise bildet die Luftströmung 5 auch ein Fördermedium, um die vom Träger 3 insbesondere durch dessen Bewegung gelöste Formulierung 2 zu dispergieren und/oder zu fördern und vorzugsweise über ein Mundstück 9 des Inhalators 1 auszutragen und an einen nicht dargestellten Benutzer auszugeben. Jedoch kann als Fördermedium für die Formulierung 2 auch ein anderer bzw. getrennter Luftstrom bzw. sonstige Luft im eingangs genannten Sinne - also auch sonstiges Gas - eingesetzt werden.

Bei der ersten Ausführungsform wird die Luftströmung 5 vorzugsweise durch das Inhalieren bzw. Einatmen des nicht dargestellten Benutzers erzeugt, und zwar insbesondere durch Ansaugen von Umgebungsluft. Der Luftstrom 5 bewirkt zunächst die Bewegung des Elements 6 und wird dann vorzugsweise auf die andere Flachseite des Trägers 3 geführt, um die durch die Bewegung des Trägers 3 vorzugsweise bereits gelöste Formulierung 2 zu dispergieren bzw. weiter zu transportieren und über das Mundstück 9 auszutragen.

Grundsätzlich kann der Träger 3 auch flexibel, flach, bandförmig, streifenförmig, blisterartig und/oder folienartig ausgebildet sein. Alternativ oder zusätzlich kann der Träger 3 auch kammerartig, kapselartig, hohlkörperartig, zylindrisch oder kugelförmig ausgebildet sein.

Fig. 2 zeigt in einem zu Fig. 1 korrespondierenden, schematischen Schnitt eine zweite Ausführungsform des vorschlagsgemäßen Zerstäubers 1. Die zweite Ausführungsform entspricht zumindest im wesentlichen der ersten Ausführungsform, so daß nachfolgend nur auf wesentliche Unterschiede gegenüber der ersten Ausführungsform eingegangen wird. Die bisherigen Ausführungen und Erläuterungen gelten insbesondere ergänzend oder entsprechend.

Bei der zweiten Ausführungsform weist der Zerstäuber 1 eine Pumpe 10 zur Erzeugung des Luftstroms 5 auf. Beim Darstellungsbeispiel wird von der Pumpe 10 Umgebungsluft angesaugt. Jedoch kann, wie bereits eingangs erläutert, auch sonstiges Gas eingesetzt werden. Der Begriff "Luft" ist daher vorzugsweise in einem entsprechend weiten Sinne zu verstehen.

Die Pumpe 10 ist beim Darstellungsbeispiel vorzugsweise manuell betätigbar bzw. antreibbar. Jedoch kann die Pumpe 10 auch auf sonstige Weise, beispielsweise elektrisch, angetrieben werden. Das Einschalten der Pumpe 10 kann beispielsweise auch durch eine Atemzugtriggerung erfolgen, so daß beim Inhalieren bzw. Einatmen die Pumpe 10 selbsttätig in Betrieb gesetzt wird, beispielsweise aufgrund eines beim ersten Einatmen entstehenden Unterdrucks, Luftstroms o. dgl.

Die Pumpe 10 kann in einem allgemeinen Sinne auch als Einrichtung zur Erzeugung und/oder Bereitstellung von unter Druck stehender Luft bzw. stehendem sonstigem Gas verstanden werden. Dementsprechend kann es sich beispielsweise auch um einen Druckluft- bzw. -gasbehälter o. dgl. - vorzugsweise mit einer entsprechenden Ventileinrichtung - handeln. Alternativ kann der Luftstrom auch auf sonstige Weise erzeugt bzw. bereitgestellt werden.

Bei der ersten und zweiten Ausführungsform bildet der Träger 3 vorzugsweise eine Begrenzung für das Element 6 in der Kammer 7 und/oder deckt die Kammer 7 mehr oder weniger ab. Jedoch ist es grundsätzlich auch möglich, daß hier eine zusätzliche Führung, Halterung, Begrenzung o. dgl. - insbesondere zur Begrenzung des Bewegungsspielraums des Elements 6 und/oder dessen Führung - eingesetzt wird bzw. werden.

Fig. 3 zeigt ein Beispiel eines Inhalators 1. Nachfolgend werden lediglich wesentliche Unterschiede gegenüber den ersten beiden Ausführungsformen erläutert, so daß die bisherigen Ausführungen und Erläuterungen insbesondere entsprechend oder ergänzend gelten.

Bei diesem Beispiel setzt der Luftstrom 5 den Träger 3 vorzugsweise unmittelbar in Bewegung, insbesondere in Schwingung. Die Schwingung erfolgt insbesondere wiederum zumindest im wesentlichen oder mit einer wesentlichen Komponente quer zur Längsrichtung bzw. Flächenerstreckung des Trägers 3.

Insbesondere wird der Luftstrom 5 an einer Flachseite zumindest im wesentlichen parallel zur Flachseite des Trägers 3 und/oder zu einem freien Ende des Trägers 3 geführt.

Bei diesem Beispiel wird der Luftstrom 5 vorzugsweise wiederum als Fördermedium zum Austragen der Formulierung 2 über das Mundstück 9 eingesetzt. Der Luftstrom 5 wird insbesondere wieder durch Inhalieren bzw. Einatmen eines nicht dargestellten Benutzers erzeugt. Jedoch ist es möglich, bei Bedarf die Pumpe 10 wie bei der zweiten Ausführungsform zur Erzeugung und/oder Unterstützung des Luftstroms 5 einzusetzen.

Es ist generell anzumerken, daß die Pumpe 10 auch nur zur Erzeugung des Luftstroms 5 verwendet werden kann, um den Träger 3 in Bewegung zu setzen. Das Fördermedium bzw. ein anderer Luftstrom kann dann dem Dispergieren und Anbringen der Formulierung 2 dienen und beispielsweise durch das Einatmen bzw. Inhalieren erzeugt werden.

Fig. 4 zeigt in einer schematischen Draufsicht eine bevorzugte Ausführungsform des Trägers 3. Der Träger 3 ist hier insbesondere endlos bzw. bandförmig oder streifenförmig und insbesondere flexibel und/oder zumindest im wesentlichen flach oder flächig ausgebildet.

Der Träger 3 kann beispielsweise aus Folie und/oder Verbundmaterial hergestellt und/oder als Blister ausgebildet sein.

Beim Darstellungsbeispiel weist der Träger 3 vorzugsweise mehrere - insbesondere in einer Reihe - angeordnete Aufnahmen 4 mit vorzugsweise jeweils einer Dosis der Formulierung 2 auf.

Jede Aufnahme 4 bzw. Dosis der Formulierung 2 ist vorzugsweise auf einem insbesondere separaten Abschnitt 11 des Trägers 3 angeordnet. Die Abschnitte 11 sind beispielsweise zungenartig ausgebildet und/oder durch Einschnitte 12 oder auf sonstige Weise voneinander getrennt, so daß sich die Abschnitte 11 insbesondere unabhängig voneinander quer zur Flächenerstreckung bewegen können. Dies erleichtert die Bewegung des Trägers 3 bzw. jeweiligen Abschnitts 11 zur Abgabe der jeweiligen Formulierung 2 im Inhalator 1.

Der Träger 3 ist vorzugsweise schrittweise durch den Inhalator 1 bzw. durch oder in einen Raum 13 des Inhalators 1 o. dgl. bewegbar, um jeweils eine Aufnahme 4 bzw. Dosis der Formulierung 2 bzw. einen Abschnitt 11 im Raum 13 in gewünschter Weise durch den Luftstrom 5 in Bewegung versetzen und die Formulierung 2 dispergieren und austragen zu können. Die Abschnitte 11 bzw. Aufnahmen 4 werden dann jeweils einzeln nacheinander vom Luftstrom 5 bewegt, so daß die gewünschte einzelweise Ausgabe der einzelnen Dosen der Formulierung 2 erfolgen kann.

Jedoch ist es grundsätzlich auch möglich, gleichzeitig mehrere Dosen und/ oder unterschiedliche Formulierungen 2 - bedarfsweise auch aus unterschiedlichen Aufnahmen 4 - auszutragen. Der Träger 3 kann hierzu beispielsweise mehrere Aufnahmen 4 mit ggf. unterschiedlichen Formulierungen 2 auf einem Abschnitt 11 aufweisen. Jedoch sind auch andere Konfigurationen möglich.

Beim Darstellungsbeispiel ist die Formulierung 2 in die einzelnen Aufnahmen 4 vorzugsweise vordosiert und insbesondere für die Lagerung hermetisch verschlossen. Das Öffnen der einzelnen Aufnahmen 4 bzw. ein Aufreißen des Trägers 3 o. dgl. erfolgt dann vorzugsweise erst unmittelbar vor oder beim Inhalieren. Bedarfsweise kann das Öffnen durch eine nicht dargestellte Öffnungseinrichtung und/oder die Bewegung des Trägers 3 - ggf. auch erst durch den Aufprall des Elements 6 - erfolgen. Bedarfsweise kann das Element 6 hierzu auch mit entsprechenden Öffnungselementen, Schneiden, Vorsprüngen o. dgl. versehen sein.

Gemäß einer nicht dargestellten Ausführungsvariante ist es möglich, den Träger 3 erst unmittelbar vor oder beim Transport in den Inhalator 1 bzw. die Kammer 13 mit der Formulierung 2 zu versehen, beispielsweise eine Aufnahme 4 zu füllen.

Gemäß einer weiteren Ausführungsvariante ist es auch möglich, die Oberfläche des Trägers 3 relativ rauh auszubilden, so daß er die vorzugsweise pulverförmige Formulierung 2 auf seiner Oberfläche aufnimmt und bei der Bewegung durch den Luftstrom 5 bzw. das Element 6 wieder in geeigneter Weise abgibt, insbesondere so daß die Formulierung 2 in erwünschter Weise in ein gasförmiges Fördermedium dispergiert und ausgetragen wird. Beispielsweise kann der Träger 3 hierbei auch fadenförmig ausgebildet sein und zur Aufnahme der Formulierung 2 beispielsweise durch ein nicht dargestelltes Reservoir mit der Formulierung 2 gefördert bzw. gezogen werden. Gemäß einer anderen Variante kann es sich bei dem Träger 3 auch um ein poröses oder vliesartiges, textilartiges oder teppichartiges Material mit insbesondere entsprechend rauher und/oder offenporiger oder offener Oberfläche handeln.

Bei den Inhalatoren gemäß Fig. 1 bis 3 ist der Träger 3 vorzugsweise von einer geeigneten, insbesondere nur schematisch angedeuteten Führungseinrichtung 14 im Raum 13 gehalten bzw. geführt, besonders bevorzugt nur an einer Seite, Kante, Ecke oder einem Ende. Bei der dritten Ausführungsform insbesondere auf der Seite, von der der Luftstrom 5 anströmt bzw. ankommt (Fig. 3 links).

Generell wird insbesondere ein Inhalator 1 zur Inhalation einer Formulierung 2 vom Träger 3 bzw. aus Aufnahmen 4 vorgeschlagen, die jeweils eine Dosis der Formulierung 2 enthalten. Die Aufnahmen 4 werden vorzugsweise jeweils dadurch entleert, daß sie von einem strömenden Gas- bzw. Luftstrom 5 direkt oder das davon bewegte Element 6 in Bewegung versetzt werden. Der Gas- bzw. Luftstroms kann durch das Einatmen eines Benutzers bzw. Patienten und/oder aktiv durch den Inhalator 1 erzeugt werden. Zur einfachen Handhabung weist der Inhalator 1 insbesondere eine Einrichtung zur insbesondere selbsttätigen Befüllung, Entleerung und/ oder Reinigung auf.

Das vorzugsweise vorgesehene Dispergierverfahren (Bewegung des Trägers 3 bzw. der geöffneten oder sich öffnenden Aufnahme 4 im Gas- bzw. Luftstrom 5 zur Ausgabe und Dispersion der Formulierung 2 im Gas- bzw. Luftstrom 5) hat verschiedene Vorteile. Es wird eine sehr gleichmäßige Dispersion mit guter Deagglomaration der Partikel ermöglicht. Es wird eine relative Flußratenunabhängigkeit ermöglicht. Insbesondere beginnt die Ausgabebewegung bzw. -vibration oder -schwingung und damit die Ausgabe der Formulierung 2 erst ab etwa einer Flußrate von 10 oder 20 l/min. Weiter kann ein für den Benutzer wahrnehmbares akustisches Signal durch die Ausgabebewegung - also beim Inhalieren - erzeugt werden.

Hinsichtlich der bisherigen und nachfolgend weiter beschriebenen Ausführungsformen des Inhalators 1 ist anzumerken, daß der Träger 3 vorzugsweise einen kanalförmigen Raum 13 - insbesondere zumindestens teilweise oder einseitig - bildet oder begrenzt, wobei der Träger 3 bzw. Raum 13 vom Luftstrom 5 durch- und/oder umströmt wird. Jedoch sind grundsätzlich auch andere Konfigurationen möglich.

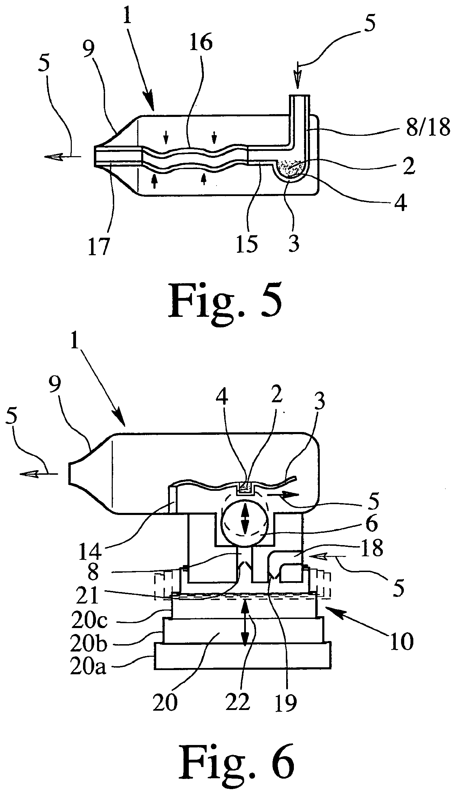

Fig. 5 zeigt in einem schematischen Schnitt ein weiteres Beispiel eines Inhalators 1. Dieses Beispiel ist grundsätzlich dem Beispiel der Fig.3 sehr ähnlich, so daß die diesbezüglichen Erläuterungen bzw. Ausführungen insbesondere entsprechend oder ergänzend gelten.

Bei diesem Beispiel schließt sich an den Träger 3 bzw. die Aufnahme 4 oder ein sonstiges Reservoir für die Formulierung 2 vorzugsweise ein zumindest bereichsweise oder einseitig flexibler und/oder bewegbarer Förderkanal 16 an, der besonders bevorzugt über einen insbesondere starren Verbindungsabschnitt 15 an den Träger 3 bzw. die Aufnahme 4, das sonstige Reservoir für die Formulierung 2 oder dgl. angeschlossen ist.

Der Förderkanal 16 ist auslaßseitig vorzugsweise mit dem Mundstück 9 verbunden, beim Darstellungsbeispiel insbesondere über einen vorzugsweise starren Ausgabekanal 17 oder dgl.

Der Förderkanal 16 ist beim Darstellungsbeispiel vorzugsweise rohrförmig und/oder im Querschnitt flach ausgebildet.

Insbesondere ist der Förderkanal 16 bzw. dessen Wandung durch flexibles, beispielsweise textilartiges oder folienartiges Material, zumindest bereichsweise, einseitig oder vollständig gebildet.

Im Gegensatz zu den anderen Ausführungsformen ist hier ein Bewegen des Trägers 3 bzw. der Aufnahme 4 zwar grundsätzlich möglich, aber nicht erforderlich. Insbesondere kann der Träger 3 bzw. die Aufnahme 4 oder ein sonstiges Reservoir für die Formulierung 2 starr bzw. unbeweglich - ggf. aber auswechselbar - ausgebildet sein, wie dies beim Darstellungsbeispiel vorzugsweise der Fall ist.

Der insbesondere beim Einatmen bzw. Inhalieren über den Zuführkanal 8 bzw. einen Einlaßkanal 18 angesaugte Luftstrom 5 bewirkt ein Lösen bzw. Fördern der vorzugsweise pulverförmigen Formulierung 2 vom Träger 3 bzw. aus der Aufnahme 4 durch den Förderkanal 16 zum Mundstück 9.

Der Luftstrom 5 bewirkt, daß der Förderkanal 16 bzw. dessen Wandung zumindest einseitig oder bereichsweise insbesondere aufgrund des Bernoulli-Effekts bewegt wird. Beim Darstellungsbeispiel erfolgt insbesondere eine flatterartige und/oder wellenartige Bewegung und/oder ein abwechselndes radiales Ausdehnen und Zusammenziehen oder -drücken des Förderkanals 16. Diese Bewegung des Förderkanals 16 kann eine wesentliche Verbesserung der Dispergierung der Formulierung 2 im Luftstrom 5 oder in einem sonstigen Fördermedium bewirken. Alternativ oder zusätzlich können so auch unterschiedliche Formulierungen 2 besonders effektiv gemischt bzw. vermischt werden.

Beim Darstellungsbeispiel wird der Förderkanal 16 vom Luftstrom 5 durchströmt. Jedoch kann der Förderkanal 16 alternativ oder zusätzlich auch vom Luftstrom 5 umströmt oder in sonstiger Weise angeströmt werden.

Anstelle einer unmittelbaren bzw. direkten Bewegung des Förderkanals 16 durch den Luftstrom 5 bzw. dessen Einwirkung kann dies mittelbar bzw. indirekt beispielsweise wiederum mittels des Elements 6, das vom Luftstrom 5 bewegt wird, erfolgen. Diesbezüglich wird auf die anderen Ausführungsformen verwiesen.

Bedarfsweise kann der Luftstrom 5 auch nur zur Bewegung des Kanalabschnitts 16 eingesetzt und ein separater Luftstrom bzw. ein separates Fördermedium zur Förderung der Formulierung 2 und dessen Austrag verwendet werden.

Es ist anzumerken, daß der Begriff "Förderkanal" vorzugsweise in einem sehr weitem Sinn zu verstehen ist. Beispielsweise kann der Förderkanal 16 an einer Seite durch eine starre Wandung begrenzt und an einer anderen Seite durch eine flexible und/oder bewegbare Wandung oder dgl. begrenzt sein, wobei der Förderkanal 16 dazwischen gebildet wird. Des weiteren sind auch beliebige Querschnittskonfigurationen möglich. Der Förderkanal 16 ist zwar vorzugsweise länglich ausgebildet und weist insbesondere einen verhältnismäßig kleiner Querschnitt oder Durchmesser im Vergleich zu seiner Länge auf, jedoch sind hier auch andere Konfigurationen und Geometrien möglich. Beispielsweise kann der Förderkanal 16 auch nur sehr kurz ausgebildet sein und/oder einen größeren Durchmesser als Länge aufweisen.

Fig. 6 zeigt eine andere Ausführungsform des vorschlagsgemäßen Inhalators 2. Diese Ausführungsform ähnelt sehr stark der zweiten Ausführungsform, so daß die diesbezüglichen Ausführungen und Erläuterungen entsprechend oder ergänzend gelten.

Bei dieser Ausführungsform ist die Pumpe 10 zur Förderung von Luft bzw. Erzeugung des Luftstroms 5 in besonders bevorzugter Weise ausgebildet.

Der Pumpe 10 ist vorzugsweise ein Einlaßkanal 18 zum Ansaugen von Umgebungsluft zugeordnet. Jedoch sind auch andere konstruktive Lösungen möglich.

Die Pumpe 10 weist vorzugsweise ein Einlaßventil 19, einen Pumpraum 20 und/oder ein Auslaßventil 21 auf.

Der Pumpraum 20 ist vorzugsweise teleskopierbar, insbesondere teleskopisch auseinanderziehbar bzw. zusammenschiebbar, wie durch den Doppelpfeil 22 in Fig. 6 angedeutet.

Beim teleskopischen Bewegen bzw. Pumpen wird abwechselnd Umgebungsluft über den Einlaßkanal 18 und das optionale Einlaßventil 19 angesaugt und über das Auslaßventil 21 und den Zuführkanal 8 abgegeben. Die Luft wird also unter Druck gesetzt, um den Luftstrom 5 zu erzeugen, insbesondere um die Formulierung 2 auszutragen und/oder eine Bewegung des Trägers 3, des Elements und/oder des Kanalabschnitts 16 zu bewirken, wie bereits oben beschrieben.

Die Pumpe 10 weist ein vorzugsweise möglichst großes Pumpvolumen bei kompaktem Aufbau auf. Aufgrund der Teleskopierbarkeit des Pumpraums 20 kann ein vergleichsweise großes Pumpvolumen erreicht werden, um dementsprechend einen starken Luftstrom 5 und dadurch eine effektive Austragung bzw. Dispergierung der Formulierung 2 bewirken zu können. Im Transportzustand kann der Pumpraum 20 zusammengeschoben werden, so daß der Inhalator 1 besonders wenig Raum beim Transport, beispielsweise in einer Tasche, einnimmt.

Vorzugsweise weist der Pumpraum 20 insbesondere äußere und/oder ringförmige Wandungs- bzw. Gehäuseteile 20a, 20b und 20c auf. Insbesondere ist der Pumpraum 20 durch mindestens zwei, drei oder mehr Gehäuseteile 20a, 20b, 20c begrenzt bzw. gebildet. Besonders bevorzugt bilden die Wandungsteile 20a, 20b, 20c äußere Gehäuseteile des Inhalators 1.

Die Wandungsteile 20a, 20b, 20c sind vorzugsweise axial ineinander schiebbar bzw. auseinander ziehbar. Fig. 6 zeigt den auseinandergezogenen Zustand. Gestrichelt ist der zusammengeschobene Zustand des Inhalators 1 bzw. Pumpraums 20 bzw. der Wandungsteile 20a, 20b, 20c dargestellt.

Besonders bevorzugt ist das äußerste und/oder unterste bzw. endseitige Wandungsteil 20a topfartig bzw. als Endstück bzw. Handhabe für einen nicht dargestellten Benutzer ausgebildet.

Besonders bevorzugt ist der Pumpraum 20 im zusammengezogenen Zustand rastend oder auf sonstige Weise arretierbar, beispielsweise für Transportzwecke.

Besonders bevorzugt ist das Pumpvolumen des Pumpraums 20 derart gewählt bzw. angepaßt, daß bei jedem Pumphub eine ausreichende Luftmenge gepumpt bzw. zur Verfügung gestellt wird, um einen vollständigen Austrag einer Dosis der Formulierung 2 bzw. eine vollständige Inhalation zu ermöglichen.

Der Pumpraum 20 ist beim Darstellungsbeispiel vorzugsweise aus mehreren, insbesondere starren Wandungsteilen 20a, 20b, 20c aufgebaut. Entsprechend ist die Wandung des Pumpraums 20 teleskopierbar. Alternativ oder zusätzlich kann der Pumpraum 20 auch balgartig bzw. aus einer flexiblen Wandung ausgebildet bzw. aufgebaut sein. Die bisherigen Ausführungen und Erläuterungen gelten dann insbesondere entsprechend. Insbesondere wird die "balgartige" Ausbildung vorzugsweise auch als "teleskopierbar" im Sinne der vorliegenden Erfindung verstanden.

Die voranstehend beschriebene Pumpe 10 bzw. der teleskopierbare Pumpraum 20 ist unabhängig von den beschriebenen Ausführungsformen insbesondere auch bei sonstigen Inhalatoren 1, Zerstäubern, Dispensern oder dgl. einsetzbar.

Beim Darstellungsbeispiel arbeitet der Inhalator besonders bevorzugt nur mechanisch. Grundsätzlich kann der Inhalator 1 jedoch auch elektrisch bzw. elektronisch arbeiten und/oder derartige Komponenten bzw. Bauteile enthalten. Dies gilt insbesondere für eine Triggereinrichtung zum Auslösen eines Austrags der Formulierung 2 oder Festlegen der Inhalationsdauer, einen Auftrieb, eine Pumpe, eine Zähleinrichtung, eine Sperre, eine Steuereinrichtung oder dergleichen.

Einzelne Merkmale und Aspekte der verschiedenen Ausführungsformen können auch beliebig miteinander kombiniert oder bei sonstigen Konstruktionen von Inhalatoren eingesetzt werden.

Die vorliegende Erfindung ist nicht auf Inhalatoren beschränkt, sondern kann entsprechend auch bei sonstigen Zerstäubern eingesetzt werden. Dementsprechend ist der Begriff "Inhalator" vorzugsweise in einem weiteren Sinne auch dahingehend zu verstehen, daß er auch sonstige Spender oder Zerstäuber, insbesondere für medizinische oder sonstige therapeutische Zwecke, umfaßt.

Nachfolgend werden bevorzugte Bestandteile und/oder Zusammensetzungen der vorzugsweise medizinischen Formulierung 2 aufgeführt. Wie bereits erwähnt, handelt es sich insbesondere um Pulver, oder um Flüssigkeiten im weitesten Sinne. Besonders bevorzugt sind in der Formulierung 2 enthalten:
Die unten genannten Verbindungen können allein oder in Kombination zur Anwendung in der erfindungsgemäßen Vorrichtung gelangen. In den unten genannten Verbindungen ist **W** einen pharmakologisch, aktiver Wirkstoff und (beispielsweise) ausgewählt aus der Gruppe bestehend aus Betamimetika, Anticholinergika, Corticosteroiden, PDE4-Inhibitoren, LTD4-Antagonisten, EGFR-Hemmern, Dopamin-Agonisten, H1-Antihistaminika, PAF-Antagonisten und PI3-Kinase Inhibitoren. Weiterhin können zwei- oder dreifach Kombinationen von **W** kombiniert werden und zur Anwendung in der erfindungsgemäßen Vorrichtung gelangen. Beispielhaft genannte Kombinationen von **W** wären:
- **W** stellt ein Betamimetika dar, kombiniert mit einem Anticholinergika, Corticosteroide, PDE4-Inhibitore, EGFR-Hemmern oder LTD4-Antagonisten,
- **W** stellt ein Anticholinergika dar, kombiniert mit einem Betamimetika, Corticosteroiden, PDE4-Inhibitoren, EGFR-Hemmern oder LTD4-Antagonisten,
- **W** stellt ein Corticosteroiden dar, kombiniert mit einem PDE4-Inhibitoren, EGFR-Hemmern oder LTD4-Antagonisten
- **W** stellt ein PDE4-Inhibitoren dar, kombiniert mit einem EGFR-Hemmern oder LTD4-Antagonisten
- **W** stellt ein EGFR-Hemmern dar, kombiniert mit einem LTD4-Antagonisten.

Als Betamimetika gelangen hierbei vorzugsweise Verbindungen zur Anwendung, die ausgewählt sind aus der Gruppe bestehend aus Albuterol, Arformoterol, Bambuterol, Bitolterol, Broxaterol, Carbuterol, Clenbuterol, Fenoterol, Formoterol, Hexoprenaline, Ibuterol, Isoetharine, Isoprenaline, Levosalbutamol, Mabuterol, Meluadrine, Metaproterenol, Orciprenaline, Pirbuterol, Procaterol, Reproterol, Rimiterol, Ritodrine, Salmefamol, Salmeterol, Soterenol, Sulphonterol, Terbutaline, Tiaramide, Tolubuterol, Zinterol, CHF-1035, HO-KU-81, KUL-1248 und
- 3-(4-{6-[2-Hydroxy-2-(4-hydroxy-3-hydroxymethyl-phenyl)-ethylamino]-hexyloxy}-butyl)-benzyl-sulfonamid
- 5-[2-(5,6-Diethyl-indan-2-ylamino)-1-hydroxy-ethyl]-8-hydroxy-1H-quinolin-2-on
- 4-Hydroxy-7-[2-{[2-{[3-(2-phenylethoxy)propyl]sulphonyl}ethyl]-amino}ethyl]-2(3H)-benzothiazolon
- 1-(2-Fluor-4-hydroxyphenyl)-2-[4-(1-benzimidazoly1)-2-methyl-2-butylamino]ethanol
- 1-[3-(4-Methoxybenzyl-amino)-4-hydroxyphenyl]-2-[4-(1-benzimidazolyl)-2-methyl-2-butylamino]ethanol
- 1-[2H-5-hydroxy-3-oxo-4H-1,4-benzoxazin-8-yl]-2-[3-(4-N,N-dimethylaminophenyl)-2-methyl-2-propylamino]ethanol
- 1-[2H-5-hydroxy-3-oxo-4H-1,4-benzoxazin-8-yl]-2-[3-(4-methoxyphenyl)-2-methyl-2-propylamino]ethanol
- 1-[2H-5-hydroxy-3-oxo-4H-1,4-benzoxazin-8-yl]-2-[3-(4-n-butyloxyphenyl)-2-methyl-2-propylamino]ethanol
- 1-[2H-5-hydroxy-3-oxo-4H-1,4-benzoxazin-8-yl]-2-{4-[3-(4-methoxyphenyl)-1,2,4-triazol-3-yl]-2-methyl-2-butylamino}ethanol
- 5-Hydroxy-8-(1-hydroxy-2-isopropylaminobutyl)-2H-1,4-benzoxazin-3-(4H)-on
- 1-(4-Amino-3-chlor-5-trifluormethylphenyl)-2-tert.-butylamino)ethanol
- 6-Hydroxy-8-{1-hydroxy-2-[2-(4-methoxy-phenyl)-1,1-dimethyl-ethylamino]-ethyl}-4H-benzo[1,4]oxazin-3-on
- 6-Hydroxy-8-{1-hydroxy-2-[2-(4-phenoxy-essigsäureethylester)-1,1-dimethyl-ethylamino]-ethyl}-4H-benzo[1,4]oxazin-3-on
- 6-Hydroxy-8-{1-hydroxy-2-[2-(4-phenoxy-essigsäure)-1,1-dimethyl-ethylamino]-ethyl}-4H-benzo[1,4]oxazin-3-on
- 8-{2-[1,1-Dimethyl-2-(2,4,6-trimethylphenyl)-ethylamino]-1-hydroxy-ethyl}-6-hydroxy-4H-benzo[1,4]oxazin-3-on
- 6-Hydroxy-8-{1-hydroxy-2-[2-(4-hydroxy-phenyl)-1,1-dimethyl-ethylamino]-ethyl}-4H-benzo[1,4]oxazin-3-on
- 6-Hydroxy-8-{1-hydroxy-2-[2-(4-isopropyl-phenyl)-1,1dimethyl-ethylamino]-ethyl}-4H-benzo[1,4]oxazin-3-on
- 8-{2-[2-(4-Ethyl-phenyl)-1,1-dimethyl-ethylamino]-1-hydroxy-ethyl}-6-hydroxy-4H-benzo[1,4]oxazin-3-on
- 8-{2-[2-(4-Ethoxy-phenyl)-1,1-dimethyl-ethylamino]-1-hydroxy-ethyl}-6-hydroxy-4H-benzo[1,4]oxazin-3-on
- 4-(4-{2-[2-Hydroxy-2-(6-hydroxy-3-oxo-3,4-dihydro-2H-benzo[1,4]oxazin-8-yl)-ethylamino]-2-methyl-propyl}-phenoxy)-buttersäure
- 8-{2-[2-(3,4-Difluor-phenyl)-1,1-dimethyl-ethylamino]-1-hydroxy-ethyl}-6-hydroxy-4H-benzo[1,4]oxazin-3-on
- 1-(4-Ethoxy-carbonylamino-3-cyano-5-fluorophenyl)-2-(tert.-butylamino)ethanol
- 2-Hydroxy-5-(1-hydroxy-2-{2-[4-(2-hydroxy-2-phenyl-ethylamino)-phenyl]-ethylamino}-ethyl)-benzaldehyd
- N-[2-Hydroxy-5-(1-hydroxy-2-{2-[4-(2-hydroxy-2-phenyl-ethylamino)-phenyl]-ethylamino}-ethyl)-phenyl]-formamid
- 8-Hydroxy-5-(1-hydroxy-2-{2-[4-(6-methoxy-biphenyl-3-ylamino)-phenyl]-ethylamino}-ethyl)-1H-quinolin-2-on
- 8-Hydroxy-5-[1-hydroxy-2-(6-phenethylamino-hexylamino)-ethyl]-1H-quinolin-2-on
- 5-[2-(2-{4-[4-(2-Amino-2-methyl-propoxy)-phenylamino]-phenyl}-ethylamino)-1-hydroxy-ethyl]-8-hydroxy-1H-quinolin-2-on
- [3-(4-{6-[2-Hydroxy-2-(4-hydroxy-3-hydroxymethyl-phenyl)-ethylamino]-hexyloxy}-butyl)-5-methyl-phenyl]-harnstoff
- 4-(2-{6-[2-(2,6-Dichloro-benzyloxy)-ethoxy]-hexylamino}-1-hydroxy-ethyl)-2-hydroxymethyl-phenol
- 3-(4-{6-[2-Hydroxy-2-(4-hydroxy-3-hydroxymethyl-phenyl)-ethylamino]-hexyloxy}-butyl)-benzylsulfonamid
- 3-(3-{7-[2-Hydroxy-2-(4-hydroxy-3-hydroxymethyl-phenyl)-ethylamino]-heptyloxy}-propyl)-benzylsulfonamid
- 4-(2-{6-[4-(3-Cyclopentanesulfonyl-phenyl)-butoxy]-hexylamino}-1-hydroxy-ethyl)-2-hydroxymethyl-phenol
- N-Adamantan-2-yl-2-(3-{2-[2-hydroxy-2-(4-hydroxy-3-hydroxymethyl-phenyl)-ethylamino]-propyl}-phenyl)-acetamid
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Anticholinergika gelangen hierbei vorzugsweise Verbindungen zur Anwendung, die ausgewählt sind aus der Gruppe bestehend aus Tiotropiumsalzen, bevorzugt das Bromidsalz, Oxitropiumsalzen, bevorzugt das Bromidsalz, Flutropiumsalzen, bevorzugt das Bromidsalz, Ipratropiumsalzen, bevorzugt das Bromidsalz, Glycopyrroniumsalzen, bevorzugt das Bromidsalz, Trospiumsalzen, bevorzugt das Chloridsalz, Tolterodin. In den vorstehend genannten Salzen stellen die Kationen die pharmakologisch aktiven Bestandteile dar. Als Anionen können die vorstehend genannten Salze bevorzugt enthalten Chlorid, Bromid, Iodid, Sulfat, Phosphat, Methansulfonat, Nitrat, Maleat, Acetat, Citrat, Fumarat, Tartrat, Oxalat, Succinat, Benzoat oder p-Toluolsulfonat, wobei Chlorid, Bromid, Iodid, Sulfat, Methansulfonat oder p-Toluolsulfonat als Gegenionen bevorzugt sind. Von allen Salzen sind die Chloride, Bromide, Iodide und Methansulfonate besonders bevorzugt.

Ebenfalls bevorzugte Anticholinergika sind ausgewählt aus den Salzen der Formel **AC-1** 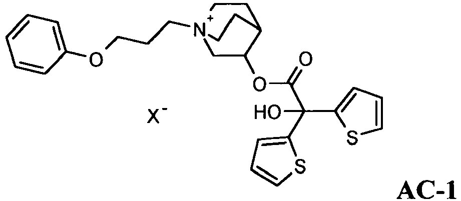 worin X⁻ ein einfach negativ geladenes Anion, bevorzugt ein Anion ausgewählt aus der Gruppe bestehend aus Fluorid, Chlorid, Bromid, Iodid, Sulfat, Phosphat, Methansulfonat, Nitrat, Maleat, Acetat, Citrat, Fumarat, Tartrat, Oxalat, Succinat, Benzoat und p-Toluolsulfonat, bevorzugt ein einfach negativ geladenes Anion, besonders bevorzugt ein Anion ausgewählt aus der Gruppe bestehend aus Fluorid, Chlorid, Bromid, Methansulfonat und p-Toluolsulfonat, insbesondere bevorzugt Bromid, bedeutet gegebenenfalls in Form ihrer Racemate, Enantiomere oder Hydrate. Von besonderer Bedeutung sind solche Arzneimittelkombinationen, die die Enantiomere der Formel **AC-1-en** 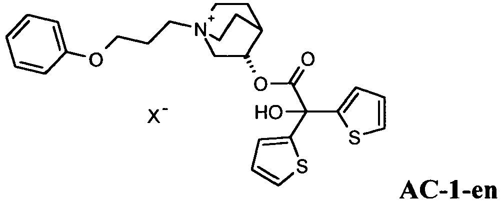 enthalten, worin X⁻ die vorstehend genannten Bedeutungen aufweisen kann. Weiterhin bevorzugte Anticholinergika sind ausgewählt aus den Salzen der Formel **AC-2** 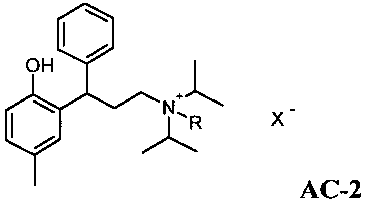 worin R entweder Methyl oder Ethyl bedeuten und worin X⁻ die vorstehend genannte Bedeutungen aufweisen kann. In einer alternativen Ausführungsform kann die Verbindung der Formel **AC-2** auch in Form der freien Base **AC-2-base** vorliegen.

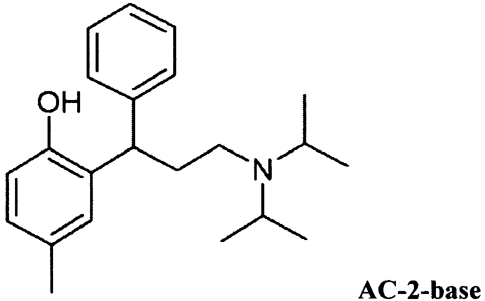

Weiterhin genannte Verbindungen sind:
- 2,2-Diphenylpropionsäuretropenolester-Methobromid
- 2,2-Diphenylpropionsäurescopinester-Methobromid
- 2-Fluor-2,2-Diphenylessigsäurescopinester-Methobromid
- 2-Fluor-2,2-Diphenylessigsäuretropenolester-Methobromid
- 3,3',4,4'-Tetrafluorbenzilsäuretropenolester-Methobromid
- 3,3',4,4'-Tetrafluorbenzilsäurescopinester-Methobromid
- 4,4'-Difluorbenzilsäuretropenolester-Methobromid
- 4,4'-Difluorbenzilsäurescopinester-Methobromid
- 3,3'-Difluorbenzilsäuretropenolester-Methobromid
- 3,3'-Difluorbenzilsäurescopinester-Methobromid
- 9-Hydroxy-fluoren-9-carbonsäuretropenolester-Methobromid
- 9-Fluor-fluoren-9-carbonsäuretropenolester-Methobromid
- 9-Hydroxy-fluoren-9-carbonsäurescopinester-Methobromid
- 9-Fluor-fluoren-9-carbonsäurescopinester-Methobromid
- 9-Methyl-fluoren-9-carbonsäuretropenolester-Methobromid
- 9-Methyl-fluoren-9-carbonsäurescopinester-Methobromid
- Benzilsäurecyclopropyltropinester-Methobromid
- 2,2-Diphenylpropionsäurecyclopropyltropinester-Methobromid
- 9-Hydroxy-xanthen-9-carbonsäurecyclopropyltropinester-Methobromid
- 9-Methyl-fluoren-9-carbonsäurecyclopropyltropinester-Methobromid
- 9-Methyl-xanthen-9-carbonsäurecyclopropyltropinester-Methobromid
- 9-Hydroxy-fluoren-9-carbonsäurecyclopropyltropinester-Methobromid
- 4,4'-Difluorbenzilsäuremethylestercyclopropyltropinester-Methobromid
- 9-Hydroxy-xanthen-9-carbonsäuretropenolester-Methobromid
- 9-Hydroxy-xanthen-9-carbonsäurescopinester-Methobromid
- 9-Methyl-xanthen-9-carbonsäuretropenolester-Methobromid
- 9-Methyl-xanthen-9-carbonsäurescopinester-Methobromid
- 9-Ethyl-xanthen-9-carbonsäuretropenolester-Methobromid
- 9-Difluormethyl-xanthen-9-carbonsäuretropenolester-Methobromid
- 9-Hydroxymethyl-xanthen-9-carbonsäurescopinester-Methobromid

Die vorstehend genannten Verbindungen sind im Rahmen der vorliegenden Erfindung auch als Salze einsetzbar, in denen statt des Methobromids, die Salze Metho-X zur Anwendung gelangen, wobei X die vorstehend für X⁻ genannten Bedeutungen haben kann.

Als Corticosteroide gelangen hierbei vorzugsweise Verbindungen zur Anwendung, die ausgewählt sind aus der Gruppe bestehend aus Beclomethason, Betamethason, Budesonid, Butixocort, Ciclesonid, Deflazacort, Dexamethason, Etiprednol, Flunisolid, Fluticason, Loteprednol, Mometason, Prednisolon, Prednison, Rofleponid, Triamcinolon, RPR-106541, NS-126, ST-26 und
- 6,9-Difluor-17-[(2-furanylcarbonyl)oxy]-11-hydroxy-16-methyl-3-oxo-androsta-1,4-dien-17-carbothionsäure (S)-fluoromethylester
- 6,9-Difluor-11-hydroxy-16-methyl-3-oxo-17-propionyloxy-androsta-1,4-dien-17-carbothionsäure (S)-(2-oxo-tetrahydro-furan-3S-yl)ester,
- 6α,9α-difluoro-11β-hydroxy-16α-methyl-3-oxo-17α-(2,2,3,3-tertamethylcyclopropylcarbonyl)oxy-androsta-1,4-diene-17β-carbonsäure cyanomethyl ester
gegebenenfalls in Form ihrer Racemate, Enantiomere oder Diastereomere und gegebenenfalls in Form ihrer Salze und Derivate, ihrer Solvate und/oder Hydrate. Jede Bezugnahme auf Steroide schließt eine Bezugnahme auf deren gegebenenfalls existierende Salze oder Derivate, Hydrate oder Solvate mit ein. Beispiele möglicher Salze und Derivate der Steroide können sein: Alkalisalze, wie beispielsweise Natrium- oder Kaliumsalze, Sulfobenzoate, Phosphate, Isonicotinate, Acetate, Dichloroacetate, Propionate, Dihydrogenphosphate, Palmitate, Pivalate oder auch Furoate.

Als PDE4-Inhibitoren gelangen hierbei vorzugsweise Verbindungen zur Anwendung, die ausgewählt sind aus der Gruppe bestehend aus Enprofyllin, Theophyllin, Roflumilast, Ariflo (Cilomilast), Tofimilast, Pumafentrin, Lirimilast, Arofyllin, Atizoram, D-4418, Bay-198004, BY343, CP-325,366, D-4396 (Sch-351591), AWD-12-281 (GW-842470), NCS-613, CDP-840, D-4418, PD-168787, T-440, T-2585, V-11294A, Cl-1018, CDC-801, CDC-3052, D-22888, YM-58997, Z-15370 und
- N-(3,5-Dichloro-1-oxo-pyridin-4-yl)-4-difluormethoxy-3-cyclopropylmethoxybenzamid
- (-)p-[(4*a*R*, 10*bS**)-9-Ethoxy-1,2,3,4,4a,10b-hexahydro-8-methoxy-2-methylbenzo[s][1,6]naphthyridin-6-yl]-N,N-diisopropylbenzamid
- (R)-(+)-1-(4-Brombenzyl)-4-[(3-cyclopentyloxy)-4-methoxyphenyl]-2-pyrrolidon
- 3-(Cyclopentyloxy-4-methoxyphenyl)-1-(4-N'-[N-2-cyano-S-methyl-isothioureido]benzyl)-2-pyrrolidon
- cis[4-Cyano-4-(3-cyclopentyloxy-4-methoxyphenyl)cyclohexan-1-carbonsäure]
- 2-carbomethoxy-4-cyano-4-(3-cyclopropylmethoxy-4-difluoromethoxy-phenyl)cyclohexan-1-on
- cis[4-Cyano-4-(3-cyclopropylmethoxy-4-difluormethoxyphenyl)cyclohexan-1-ol]
- (R)-(+)-Ethyl[4-(3-cyclopentyloxy-4-methoxyphenyl)pyrrolidin-2-yliden]acetat
- (S)-(-)-Ethyl[4-(3-cyclopentyloxy-4-methoxyphenyl)pyrrolidin-2-yliden]acetat
- 9-Cyclopentyl-5,6-dihydro-7-ethyl-3-(2-thienyl)-9*H*-pyrazolo[3,4-c]-1,2,4-triazolo[4,3-a]pyridin
- 9-Cyclopentyl-5,6-dihydro-7-ethyl-3-(*tert*-butyl)-9*H*-pyrazolo[3,4-c]-1,2,4-triazolo[4,3-a]pyridin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als LTD4-Antagonisten gelangen hierbei vorzugsweise Verbindungen zur Anwendung, die ausgewählt sind aus der Gruppe bestehend aus Montelukast, Pranlukast, Zafirlukast, MCC-847 (ZD-3523), MN-001, MEN-91507 (LM-1507), VUF-5078, VUF-K-8707, L-733321 und
- 1-(((R)-(3-(2-(6,7-Difluor-2-quinolinyl)ethenyl)phenyl)-3-(2-(2-hydroxy-2-propyl)phenyl)thio)methylcyclopropan-essigsäure,
- 1-(((1(R)-3(3-(2-(2,3-Dichlorthieno[3,2-b]pyridin-5-yI)-(E)-ethenyl)phenyl)-3-(2-(1-hydroxy-1-methylethyl)phenyl)-propyl)thio)methyl)cyclopropanessigsäure
- [2-[[2-(4-tert-Butyl-2-thiazolyl)-5-benzofuranyl]oxymethyl]phenyl]-essigsäure
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat. Unter Salzen oder Derivaten zu deren Bildung die LTD4-Antagonisten gegebenenfalls in der Lage sind, werden beispielsweise verstanden: Alkalisalze, wie beispielsweise Natrium- oder Kaliumsalze, Erdalkalisalze, Sulfobenzoate, Phosphate, Isonicotinate, Acetate, Propionate, Dihydrogenphosphate, Palmitate, Pivalate oder auch Furoate.

Als EGFR-Hemmer gelangen hierbei vorzugsweise Verbindungen zur Anwendung, die ausgewählt sind aus der Gruppe bestehend aus Cetuximab, Trastuzumab, ABX-EGF, Mab ICR-62 und
- 4-[(3-Chlor-4-fluorphenyl)amino]-6-{[4-(morpholin-4-yl)-1-oxo-2-buten-1-yl]amino}-7-cyclopropylmethoxy-chinazolin
- 4-[(3-Chlor-4-fluorphenyl)amino]-6-{[4-(N,N-diethylamino)-1-oxo-2-buten-1-yl]amino}-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopropylmethoxy-chinazolin
- 4-[(R)-(1-Phenyl-ethyl)amino]-6-{[4-(morpholin-4-yl)-1-oxo-2-buten-1-yl]amino}-7-cyclopentyloxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-cyclopropylmethoxy-chinazolin
- 4-[(3-Chlor-4-fluor-phenyl)amino]-6-{[4-((R)-6-methyl-2-oxo-morpholin-4-yl)-1-oxo-2-buten-1-yl]amino}-7-[(S)-(tetrahydrofuran-3-yl)oxy]-chinazolin
- 4-[(3-Chlor-4-fluor-phenyl)amino]-6-{[4-((R)-2-methoxymethyl-6-oxo-morpholin-4-yl)-1-oxo-2-buten-1-yl]amino}-7-cyclopropylmethoxy-chinazolin
- 4-[(3-Chlor-4-fluor-phenyl)amino]-6-[2-((S)-6-methyl-2-oxo-morpholin-4-yl)-ethoxy]-7-methoxy-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-cyclopentyloxy-chinazolin
- 4-[(R)-(1-Phenyl-ethyl)amino]-6-{[4-(N,N-bis-(2-methoxy-ethyl)-amino)-1-oxo-2-buten-1-yl]amino}-7-cyclopropylmethoxy-chinazolin
- 4-[(R)-(1-Phenyl-ethyl)amino]-6-({4-(N-(2-methoxy-ethyl)-N-ethyl-amino]-1-oxo-2-buten-1-yl}amino)-7-cyclopropylmethoxy-chinazolin
- 4-[(R)-(1-Phenyl-ethyl)amino]-6-({4-[N-(2-methoxy-ethyl)-N-methyl-amino]-1-oxo-2-buten-1-yl}amino)-7-cyclopropylmethoxy-chinazolin
- 4-[(R)-(1-Phenyl-ethyl)amino]-6-({4-(N-(tetrahydropyran-4-yl)-N-methyl-amino]-1-oxo-2-buten-1-yl}amino)-7-cyclopropylmethoxy-chinazolin
- 4-[(3-Chlor-4-fluorphenyl)amino]-6-{[4-(N,N-dimethylamino)-1-oxo-2-buten-1-yl]amino}-7-((R)-tetrahydrofuran-3-yloxy)-chinazolin
- 4-[(3-Chlor-4-fluorphenyl)amino]-6-{[4-(N,N-dimethylamino)-1-oxo-2-buten-1-yl]amino}-7-((S)-tetrahydrofuran-3-yloxy)-chinazolin
- 4-[(3-Chlor-4-fluorphenyl)amino]-6-({4-[N-(2-methoxy-ethyl)-N-methyl-amino]-1-oxo-2-buten-1-yl}amino)-7-cyclopentyloxy-chinazolin
- 4-[(3-Chlor-4-fluorphenyl)amino]-6-{[4-(N-cyclopropyl-N-methyl-amino)-1-oxo-2-buten-1-yl]amino}-7-cyclopentyloxy-chinazolin
- 4-[(3-Chlor-4-fluorphenyl)amino]-6-{[4-(N,N-dimethylamino)-1-oxo-2-buten-1-yl]amino}-7-[(R)-(tetrahydrofuran-2-yl)methoxy]-chinazolin
- 4-[(3-Chlor-4-fluorphenyl)amino]-6-{[4-(N,N-dimethylamino)-1-oxo-2-buten-1-yl]amino}-7-[(S)-(tetrahydrofuran-2-yl)methoxy]-chinazolin
- 4-[(3-Ethinyl-phenyl)amino]-6,7-bis-(2-methoxy-ethoxy)-chinazolin
- 4-[(3-Chlor-4-fluorphenyl)amino]-7-[3-(morpholin-4-yl)-propyloxy]-6-[(vinylcarbonyl)amino]-chinazolin
- 4-[(R)-(1-Phenyl-ethyl)amino]-6-(4-hydroxy-phenyl)-7H-pyrrolo[2,3-d]pyrimidin
- 3-Cyano-4-[(3-chlor-4-fluorphenyl)amino]-6-{[4-(N,N-dimethylamino)-1-oxo-2-buten-1-yl]amino}-7-ethoxy-chinolin
- 4-{[3-Chlor-4-(3-fluor-benzyloxy)-phenyl]amino}-6-(5-{[(2-methansulfonyl-ethyl)amino]methyl}-furan-2-yl)chinazolin
- 4-[(R)-(1-Phenyl-ethyl)amino]-6-{[4-((R)-6-methyl-2-oxo-morpholin-4-yl)-1-oxo-2-buten-1-yl]amino}-7-methoxy-chinazolin
- 4-[(3-Chlor-4-fluorphenyl)amino]-6-{[4-(morpholin-4-yl)-1-oxo-2-buten-1-yl]amino}-7-[(tetrahydrofuran-2-yl)methoxy]-chinazolin
- 4-[(3-Chlor-4-fluorphenyl)amino]-6-({4-[N,N-bis-(2-methoxy-ethyl)-amino]-1-oxo-2-buten-1-yl}amino)-7-[(tetrahydrofuran-2-yl)methoxy]-chinazolin
- 4-[(3-Ethinyl-phenyl)amino]-6-{[4-(5,5-dimethyl-2-oxo-morpholin-4-yl)-1-oxo-2-buten-1-yl]amino}-chinazolin
- 4-[(3-Chlor-4-fluor-phenyl)amino]-6-[2-(2,2-dimethyl-6-oxo-morpholin-4-yl)-ethoxy]-7-methoxy-chinazolin
- 4-[(3-Chlor-4-fluor-phenyl)amino]-6-[2-(2,2-dimethyl-6-oxo-morpholin-4-yl)-ethoxy]-7-[(R)-(tetrahydrofuran-2-yl)methoxy]-chinazolin
- 4-[(3-Chlor-4-fluor-phenyl)amino]-7-[2-(2,2-dimethyl-6-oxo-morpholin-4-yl)-ethoxy]-6-[(S)-(tetrahydrofuran-2-yl)methoxy]-chinazolin
- 4-[(3-Chlor-4-fluor-phenyl)amino]-6-{2-[4-(2-oxo-morpholin-4-yl)-piperidin-1-yl]-ethoxy}-7-methoxy-chinazolin
- 4-[(3-Chlor-4-fluor-phenyl)amino]-6-[1-(tert.-butyloxycarbonyl)-piperidin-4-yloxy]-7-methoxy-chinazolin
- 4-[(3-Chlor-4-fluor-phenyl)amino]-6-(trans-4-amino-cyclohexan-1-yloxy)-7-methoxy-chinazolin
- 4-[(3-Chlor-4-fluor-phenyl)amino]-6-(trans-4-methansulfonylamino-cyclohexan-1-yloxy)-7-methoxy-chinazolin
- 4-[(3-Chlor-4-fluor-phenyl)amino]-6-(tetrahydropyran-3-yloxy)-7-methoxy-chinazolin
- 4-[(3-Chlor-4-fluor-phenyl)amino]-6-(1-methyl-piperidin-4-yloxy)-7-methoxy-chinazolin
- 4-[(3-Chlor-4-fluor-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(methoxymethyl)carbonyl]-piperidin-4-yloxy}-7-methoxy-chinazolin
- 4-[(3-Chlor-4-fluor-phenyl)amino]-6-(piperidin-3-yloxy)-7-methoxy-chinazolin
- 4-[(3-Chlor-4-fluor-phenyl)amino]-6-[1-(2-acetylamino-ethyl)-piperidin-4-yloxy]-7-methoxy-chinazolin
- 4-[(3-Chlor-4-fluor-phenyl)amino]-6-(tetrahydropyran-4-yloxy)-7-ethoxy-chinazolin
- 4-[(3-Chlor-4-fluor-phenyl)amino]-6-((S)-tetrahydrofuran-3-yloxy)-7-hydroxy-chinazolin
- 4-[(3-Chlor-4-fluor-phenyl)amino]-6-(tetrahydropyran-4-yloxy)-7-(2-methoxy-ethoxy)-chinazolin
- 4-[(3-Chlor-4-fluor-phenyl)amino]-6-{trans-4-[(dimethylamino)sulfonylamino]-cyclohexan-1-yloxy}-7-methoxy-chinazolin
- 4-[(3-Chlor-4-fluor-phenyl)amino]-6-{trans-4-[(morpholin-4-yl)carbonylamino]-cyclohexan-1-yloxy}-7-methoxy-chinazolin
- 4-[(3-Chlor-4-fluor-phenyl)amino]-6-{trans-4-[(morpholin-4-yl)sulfonylamino]-cyclohexan-1-yloxy}-7-methoxy-chinazolin
- 4-[(3-Chlor-4-fluor-phenyl)amino]-6-(tetrahydropyran-4-yloxy)-7-(2-acetylamino-ethoxy)-chinazolin
- 4-[(3-Chlor-4-fluor-phenyl)amino]-6-(tetrahydropyran-4-yloxy)-7-(2-methansulfonylamino-ethoxy)-chinazolin
- 4-[(3-Chlor-4-fluor-phenyl)amino]-6-{1-[(piperidin-1-yl)carbonyl]-piperidin-4-yloxy}-7-methoxy-chinazolin
- 4-[(3-Chlor-4-fluor-phenyl)amino]-6-(1-aminocarbonylmethyl-piperidin-4-yloxy)-7-methoxy-chinazolin
- 4-[(3-Chlor-4-fluor-phenyl)amino]-6-(cis-4-{N-[(tetrahydropyran-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(morpholin-4-yl)sulfonyl]-N-methyl-amino}cyclohexan-1-yloxy)-7-methoxy-chinazolin
- 4-[(3-Chlor-4-fluor-phenyl)amino]-6-(trans-4-ethansulfonylamino-cyclohexan-1-yloxy)-7-methoxy-chinazolin
- 4-[(3-Chlor-4-fluor-phenyl)amino]-6-(1-methansulfonyl-piperidin-4-yloxy)-7-ethoxy-chinazolin
- 4-[(3-Chlor-4-fluor-phenyl)amino]-6-(1-methansulfonyl-piperidin-4-yloxy)-7-(2-methoxy-ethoxy)-chinazolin
- 4-[(3-Chlor-4-fluor-phenyl)amino]-6-[1-(2-methoxy-acetyl)-piperidin-4-yloxy]-7-(2-methoxy-ethoxy)-chinazolin
- 4-[(3-Chlor-4-fluor-phenyl)amino]-6-(cis-4-acetylamino-cyclohexan-1-yloxy)-7-methoxy-chinazolin
- 4-[(3-Ethinyl-phenyl)amino]-6-[1-(tert.-butyloxycarbonyl)-piperidin-4-yloxy]-7-methoxy-chinazolin
- 4-[(3-Ethinyl-phenyl)amino]-6-(tetrahydropyran-4-yloxy]-7-methoxy-chinazolin
- 4-[(3-Chlor-4-fluor-phenyl)amino]-6-(cis-4-{N-[(piperidin-1-yl)carbonyl]-N-methyl-amino}-cyclohexan-1-yloxy)-7-methoxy-chinazolin
- 4-[(3-Chlor-4-fluor-phenyl)amino]-6-(cis-4-{N-[(4-methyl-piperazin-1-yl)carbonyl]-N-methyl-amino}-cyclohexan-1-yloxy)-7-methoxy-chinazolin
- 4-[(3-Chlor-4-fluor-phenyl)amino]-6-{cis-4-[(morpholin-4-yl)carbonylamino]-cyclohexan-1-yloxy}-7-methoxy-chinazolin
- 4-[(3-Chlor-4-fluor-phenyl)amino]-6-{1-[2-(2-oxopyrrolidin-1-yl)ethyl]-piperidin-4-yloxy}-7-methoxy-chinazolin
- 4-[(3-Chlor-4-fluor-phenyl)amino]-6-{1-[(morpholin-4-yl)carbonyl]-piperidin-4-yloxy}-7-(2-methoxy-ethoxy)-chinazolin
- 4-[(3-Ethinyl-phenyl)amino]-6-(1-acetyl-piperidin-4-yloxy)-7-methoxy-chinazolin
- 4-[(3-Ethinyl-phenyl)amino]-6-(1-methyl-piperidin-4-yloxy)-7-methoxy-chinazolin
- 4-[(3-Ethinyl-phenyl)amino]-6-(1-methansulfonyl-piperidin-4-yloxy)-7-methoxy-chinazolin
- 4-[(3-Chlor-4-fluor-phenyl)amino]-6-(1-methyl-piperidin-4-yloxy)-7(2-methoxy-ethoxy)-chinazolin
- 4-[(3-Chlor-4-fluor-phenyl)amino]-6-(1-isopropyloxycarbonyl-piperidin-4-yloxy)-7-methoxy-chinazolin
- 4-[(3-Chlor-4-fluor-phenyl)amino]-6-(cis-4-methylamino-cyclohexan-1-yloxy)-7-methoxy-chinazolin
- 4-[(3-Chlor-4-fluor-phenyl)amino]-6-{cis-4-[N-(2-methoxy-acetyl)-N-methyl-amino]-cyclohexan-1-yloxy}-7-methoxy-chinazolin
- 4-[(3-Ethinyl-phenyl)amino]-6-(piperidin-4-yloxy)-7-methoxy-chinazolin
- 4-[(3-Ethinyl-phenyl)amino]-6-[1-(2-methoxy-acetyl)-piperidin-4-yloxy]-7-methoxy-chinazolin
- 4-[(3-Ethinyl-phenyl)amino]-6-{1-[(morpholin-4-yl)carbonyl]-piperidin-4-yloxy}-7-methoxy-chinazolin
- 4-[(3-Chlor-4-fluor-phenyl)amino]-6-{1-[(cis-2,6-dimethyl-morpholin-4-yl)carbonyl]-piperidin-4-yloxy}-7-methoxy-chinazolin
- 4-[(3-Chlor-4-fluor-phenyl)amino]-6-{1-[(2-methyl-morpholin-4-yl)carbonyl]-piperidin-4-yloxy}-7-methoxy-chinazolin
- 4-[(3-Chlor-4-fluor-phenyl)amino]-6-{1-[(S,S)-(2-oxa-5-aza-bicyclo[2.2.1]hept-5-yl)carbonyl]-piperidin-4-yloxy}-7-methoxy-chinazolin
- 4-[(3-Chlor-4-fluor-phenyl)amino]-6-{1-[(N-methyl-N-2-methoxyethyl-amino)carbonyl]-piperidin-4-yloxy}-7-methoxy-chinazolin
- 4-[(3-Chlor-4-fluor-phenyl)amino]-6-(1-ethyl-piperidin-4-yloxy)-7-methoxy-chinazolin
- 4-[(3-Chlor-4-fluor-phenyl)amino]-6-{1-[(2-methoxyethyl)carbonyl]-piperidin-4-yloxy}-7-methoxy-chinazolin
- 4-[(3-Chlor-4-fluor-phenyl)amino]-6-{1-[(3-methoxypropyl-amino)-carbonyl]-piperidin-4-yloxy}-7-methoxy-chinazolin
- 4-[(3-Chlor-4-fluor-phenyl)amino]-6-[cis-4-(N-methansulfonyl-N-methyl-amino)-cyclohexan-1-yloxy]-7-methoxy-chinazolin
- 4-[(3-Chlor-4-fluor-phenyl)amino]-6-[cis-4-(N-acetyl-N-methyl-amino)-cyclohexan-1-yloxy]-7-methoxy-chinazolin
- 4-[(3-Chlor-4-fluor-phenyl)amino]-6-(trans-4-methylamino-cyclohexan-1-yloxy)-7-methoxy-chinazolin
- 4-[(3-Chlor-4-fluor-phenyl)amino]-6-[trans-4-(N-methansulfonyl-N-methyl-amino)-cyclohexan-1-yloxy]-7-methoxy-chinazolin
- 4-[(3-Chlor-4-fluor-phenyl)amino]-6-(trans-4-dimethylamino-cyclohexan-1-yloxy)-7-methoxy-chinazolin
- 4-[(3-Chlor-4-fluor-phenyl)amino]-6-(trans-4- {N-[(morpholin-4-yl)carbonyl]-N-methyl-amino}-cyclohexan-1-yloxy)-7-methoxy-chinazolin
- 4-[(3-Chlor-4-fluor-phenyl)amino]-6-[2-(2,2-dimethyl-6-oxo-morpholin-4-yl)-ethoxy]-7-[(S)-(tetrahydrofuran-2-yl)methoxy]-chinazolin
- 4-[(3-Chlor-4-fluor-phenyl)amino]-6-(1-methansulfonyl-piperidin-4-yloxy)-7-methoxy-chinazolin
- 4-[(3-Chlor-4-fluor-phenyl)amino]-6-(1-cyano-piperidin-4-yloxy)-7-methoxy-chinazolin
gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als Dopamin-Agonisten gelangen hierbei vorzugsweise Verbindungen zur Anwendung, die ausgewählt sind aus der Gruppe bestehend aus Bromocriptin, Cabergolin, Alpha-Dihydroergocryptin, Lisurid, Pergolid, Pramipexol, Roxindol, Ropinirol, Talipexol, Tergurid und Viozan, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Als H1-Antihistaminika gelangen hierbei vorzugsweise Verbindungen zur Anwendung, die ausgewählt sind aus der Gruppe bestehend aus Epinastin, Cetirizin, Azelastin, Fexofenadin, Levocabastin, Loratadin, Mizolastin, Ketotifen, Emedastin, Dimetinden, Clemastin, Bamipin, Cexchlorpheniramin, Pheniramin, Doxylamin, Chlorphenoxamin, Dimenhydrinat, Diphenhydramin, Promethazin, Ebastin, Desloratidin und Meclozin, gegebenenfalls in Form ihrer Racemate, Enantiomere, Diastereomere und gegebenenfalls in Form ihrer pharmakologisch verträglichen Säureadditionssalze, Solvate oder Hydrate. Erfindungsgemäß bevorzugt sind die Säureadditionssalze der Betamimetika ausgewählt aus der Gruppe bestehend aus Hydrochlorid, Hydrobromid, Hydroiodid, Hydrosulfat, Hydrophosphat, Hydromethansulfonat, Hydronitrat, Hydromaleat, Hydroacetat, Hydrocitrat, Hydrofumarat, Hydrotartrat, Hydrooxalat, Hydrosuccinat, Hydrobenzoat und Hydro-p-toluolsulfonat.

Außerdem können inhalierbare Makromoleküle verwendet werden, wie in EP 1 003 478 A1 oder CA 2297174 A1 offenbart.

Weiterhin kann die Verbindung aus der Gruppe der Derivate von Mutterkornalkaloiden, der Triptane, der CGRP-Hemmern, der Phosphodiesterase-V-Hemmer stammen, gegebenenfalls in Form ihrer Racemate, Enantiomere oder Diastereomere, gegebenenfalls in Form ihrer pharmakologisch verträglichen Säureadditionssalze, ihrer Solvate und/oder Hydrate.

Als Derivate der Mutterkornalkaloide: Dihydroergotamin, Ergotamin.

**Bezugszeichenliste**

| | | | |
|---|---|---|---|
| 1 | Inhalator | 13 | Raum |
| 2 | Formulierung | 14 | Führung |
| 3 | Träger | 15 | Verbindungsabschnitt |
| 4 | Aufnahme | 16 | Förderkanal |
| 4a | Abdeckung | 17 | Ausgabekanal |
| 5 | Luftstrom | 18 | Einlaßkanal |
| 6 | Element | 19 | Einlaßventil |
| 7 | Kammer | 20 | Pumpraum |
| 8 | Zuführkanal | 20a | Wandungsteil |
| 9 | Mundstück | 20b | Wandungsteil |
| 10 | Pumpe | 20c | Wandungsteil |
| 11 | Abschnitt | 21 | Auslaßventil |
| 12 | Einschnitt | 22 | Doppelpfeil |

## Patentansprüche

1. Inhalator (1) mit einem Träger (3) zur Inhalation einer Formulierung (2) von dem Träger (3), wobei zum oder beim Austrag und/oder Dispergieren der Formulierung (2) zumindest ein Teil des Trägers (3) durch einen Luftstrom (5) bewegbar, insbesondere in Schwingung versetzbar, ist,
**dadurch gekennzeichnet,**
**daß** der Inhalator (1) ein vom Träger (3) getrenntes Element (6) aufweist, das von dem Luftstrom (5) zur Bewegung zumindest eines Teils des Trägers (3) bewegbar ist, wobei das Element (6) lose in einer insbesondere von dem Träger (3) begrenzten Kammer (7) aufgenommen ist, die vorzugsweise von dem Luftstrom (5) durchströmbar ist.

2. Inhalator (1) nach Anspruch 1, **dadurch gekennzeichnet,**
**daß** der Träger (3) flexibel, flach, bandförmig, streifenförmig, blisterartig und/oder folienartig ausgebildet ist.

3. Inhalator nach Anspruch 1 oder 2, **dadurch gekennzeichnet, daß** das Element (6) quer zur Längs- oder Flächenerstreckung auf den Träger (3) trifft, insbesondere auf einer der Formulierung (2) abgewandten Trägerseite.

4. Inhalator nach einem der voranstehenden Ansprüche, **dadurch gekennzeichnet, daß** der Träger (3) endlos ausgebildet ist und/oder mehrere Dosen der Formulierung (2) aufweist, die nacheinander austragbar sind.

5. Inhalator nach einem der voranstehenden Ansprüche, **dadurch gekennzeichnet, daß** der Träger (3) schrittweise oder abschnittsweise in einen vom Luftstrom (5) angeströmten Bereich oder Raum (13) bewegbar oder förderbar ist.

6. Inhalator nach Anspruch 5, **dadurch gekennzeichnet, daß** der Träger (3) mehrere Aufnahmen (4) mit jeweils einer Dosis der Formulierung (2) aufweist, wobei jede Aufnahme auf einem separaten Abschnitt (11) angeordnet ist und die Abschnitte (11) zungenartig ausgebildet und/oder durch Einschnitte (12) voneinander getrennt sind.

7. Inhalator (1) mit einem Förderkanal (16) zur Inhalation einer Formulierung (2) durch einen Förderkanal (16), wobei zum oder beim Austrag und/oder Dispergieren der Formulierung (2) zumindest ein Teil des Förderkanals (16) durch einen Luftstrom (5) bewegbar, insbesondere in Schwingung versetzbar, ist,
**dadurch gekennzeichnet,**
**daß** der Inhalator (1) ein vom Förderkanal (16) getrenntes Element (6) aufweist, das von dem Luftstrom (5) zur Bewegung zumindest eines Teils des Förderkanals (16) bewegbar ist, wobei das Element (6) lose in einer begrenzten Kammer (7) aufgenommen ist, die vorzugsweise von dem Luftstrom (5) durchströmbar ist.

8. Inhalator (1) nach Anspruch 7, **dadurch gekennzeichnet, daß** der Förderkanal (16) zumindest bereichsweise oder einseitig flexibel ausgebildet ist.

9. Inhalator nach einem der voranstehenden Ansprüche, **dadurch gekennzeichnet, daß** das Element (6) vom Luftstrom (5) in Schwingung versetzbar ist.

10. Inhalator nach einem der Ansprüche 1 bis 6, **dadurch gekennzeichnet, daß** das Element (6) schlagend auf den Träger (3) zum Austrag und/oder Dispergieren der Formulierung (2) einwirkt.

11. Inhalator nach einem der voranstehenden Ansprüche, **dadurch gekennzeichnet, daß** das Element (6) kugelförmig oder zylindrisch ausgebildet ist.

12. Inhalator nach einem der voranstehenden Ansprüche, **dadurch gekennzeichnet, daß** der Luftstrom (5) das Element (6) in Bewegungsrichtung anströmt.

13. Inhalator nach einem der voranstehenden Ansprüche, **dadurch gekennzeichnet, daß** der Luftstrom (5) durch Einatmen bzw. Inhalieren erzeugbar oder auslösbar ist.

14. Inhalator nach einem der Ansprüche 1 bis 12, **dadurch gekennzeichnet, daß** der Inhalator (1) eine Pumpe (10) zur Erzeugung des Luftstroms (5) aufweist.

15. Inhalator (1) nach Anspruch 14, **dadurch gekennzeichnet, daß** die Pumpe (10) einen teleskopierbaren Pumpraum (20) aufweist.

## Claims

1. Inhaler (1) with a carrier (3) for inhaling a formulation (2) from the carrier (3), wherein for the purpose of or during the expulsion and/or dispersion of the formulation (2) at least part of the carrier (3) can be moved, particularly set vibrating, by an air current (5),
**characterised in that**
the inhaler (1) has an element (6) separate from the carrier (3), the element being movable by the air current (5) in order to move at least part of the carrier (3),
wherein the element (6) is loosely held in a chamber (7) which is delimited in particular by the carrier (3) and through which the air current (5) can preferably flow.

2. Inhaler (1) according to claim 1, **characterised in that** the carrier (3) is flexible, flat, in the form of a ribbon, a strip, a blister and/or a foil.

3. Inhaler according claims 1 or 2, **characterised in that** the element (6) strikes the carrier (3) at right angles to the longitudinal or surface direction, particularly on a side of the carrier remote from the formulation (2).

4. Inhaler according to one of the preceding claims, **characterised in that** the carrier (3) is of endless construction and/or contains several doses of the formulation (2) which can be expelled one after another.

5. Inhaler according to one of the preceding claims, **characterised in that** the carrier (3) can be moved or conveyed stepwise or by degrees into a region or chamber (13) onto which the air current (5) flows.

6. Inhaler according to claim 5, **characterised in that** the carrier (3) comprises several receptacles (4), each containing one dose of the formulation (2), wherein each receptacle (4) is arranged on a separate portion (11) and the portions (11) are shaped like tongues and/or are separated from one another by cuts (12).

7. Inhaler (1) with a delivery channel (16) for inhaling a formulation (2) through a delivery channel (16), wherein for the purpose of or during the expulsion and/or dispersion of the formulation (2) at least part of the delivery channel (16) can be moved, particularly set vibrating, by an air current (5),
**characterised in that**
the inhaler (1) has an element (6) separate from the delivery channel (16), the element being movable by the air current (5) in order to move at least part of the delivery channel (16), wherein the element (6) is loosely held in a limited chamber (7), through which the air current (5) can preferably flow.

8. Inhaler (1) according to claim 7, **characterised in that** the delivery channel (16) is flexible at least in parts or on one side.

9. Inhaler according one of the preceding claims, **characterised in that** the element (6) can be set vibrating by the air current (5).

10. Inhaler according to one of claims 1 to 6, **characterised in that** the element (6) has the effect of striking the carrier (3) in order to expel and/or disperse the formulation (2).

11. Inhaler according to one of the preceding claims, **characterised in that** the element (6) is spherical or cylindrical in construction.

12. Inhaler according to one of the preceding claims, **characterised in that** the air current (5) flows onto the element (6) in the direction of movement.

13. Inhaler according to one of the preceding claims, **characterised in that** the air current (5) can be generated or initiated by breathing in or inhaling.

14. Inhaler according to one of claims 1 to 12, **characterised in that** the inhaler (1) comprises a pump (10) for producing the air current (5).

15. Inhaler (1) according to claim 14, **characterised in that** the pump (10) has a telescopic pump chamber (20).

## Revendications

1. Inhalateur (1) comportant un support (3) servant à l'inhalation d'une formulation (2) provenant du support (3), dans lequel aux fins ou lors de la décharge et/ou de la dispersion de la formulation (2) au moins une partie du support (3) peut être déplacée par un flux d'air (5), notamment peut être amenée en vibration,
**caractérisé en ce**
**que** l'inhalateur (1) présente un élément (6) séparé du support (3), qui peut être déplacé par le flux d'air (5) pour déplacer au moins une partie du support (3), dans lequel l'élément (6) est logé de manière lâche dans une chambre (7) délimitée notamment par le support (3), qui peut être traversée de préférence par le flux d'air (5).

2. Inhalateur (1) selon la revendication 1, **caractérisé en ce**
**que** le support (3) est réalisé de manière flexible, plate, en forme de bande, en forme de ruban, à la manière d'un blister et/ou à la manière d'un film.

3. Inhalateur selon la revendication 1 ou 2, **caractérisé en ce que** l'élément (6) arrive sur le support (3) de manière transversale par rapport à l'extension longitudinale ou en surface, notamment sur un côté de support éloigné de la formulation (2).

4. Inhalateur selon l'une des revendications précédentes, **caractérisé en ce que** le support (3) est réalisé sans fin et/ou présente plusieurs doses de la formulation (2), qui peuvent être déchargées les unes après les autres.

5. Inhalateur selon l'une des revendications précédentes, **caractérisé en ce que** le support (3) peut être déplacé ou convoyé progressivement ou par sections dans une zone ou un espace (13) parcouru par le flux d'air (5).

6. Inhalateur selon la revendication 5, **caractérisé en ce que** le support (3) présente plusieurs logements (4) ayant chacun une dose de la formulation (2), dans lequel chaque logement est disposé sur une section (11) distincte et les sections (11) sont réalisées à la manière de languettes et/ou sont séparées les unes des autres par des entailles (12).

7. Inhalateur (1) comportant un canal de convoyage (16) servant à l'inhalation d'une formulation (2) par un canal de convoyage (16), dans lequel aux fins de ou lors de la décharge et/ou de la dispersion de la formulation (2), au moins une partie du canal de convoyage (16) peut être déplacée par un flux d'air (5), notamment peut être amenée en vibration,
**caractérisé en ce**
**que** l'inhalateur (1) présente un élément (6) séparé du canal de convoyage (16), qui peut être déplacé par le flux d'air (5) pour déplacer au moins une partie du canal de convoyage (16), dans lequel l'élément (6) est logé de manière lâche dans une chambre (7) délimitée, qui peut être traversée de préférence par le flux d'air (5).

8. Inhalateur (1) selon la revendication 7, **caractérisé en ce que** le canal de convoyage (16) est réalisé de manière flexible au moins par zones ou sur un seul côté.

9. Inhalateur selon l'une des revendications précédentes, **caractérisé en ce que** l'élément (6) peut être amené en vibration par le flux d'air (5).

10. Inhalateur selon l'une des revendications 1 à 6, **caractérisé en ce que** l'élément (6) agit avec percussion sur le support (3) aux fins de la décharge et/ou de la dispersion de la formulation (2).

11. Inhalateur selon l'une des revendications précédentes, **caractérisé en ce que** l'élément (6) est réalisé en forme de sphère ou de manière cylindrique.

12. Inhalateur selon l'une des revendications précédentes, **caractérisé en ce que** le flux d'air (5) parcourt l'élément (6) dans la direction de déplacement.

13. Inhalateur selon l'une des revendications précédentes, **caractérisé en ce que** le flux d'air (5) peut être généré ou déclenché par une inspiration ou inhalation.

14. Inhalateur selon l'une des revendications 1 à 12, **caractérisé en ce que** l'inhalateur (1) présente une pompe (10) servant à générer le flux d'air (5).

15. Inhalateur (1) selon la revendication 14, **caractérisé en ce que** la pompe (10) présente un espace de pompage (20) télescopique.
